# EUROPÄISCHE PATENTANMELDUNG

(11) **EP 1 308 176 A2**
(43) Veröffentlichungstag der Anmeldung: **07.05.2003**
(21) Anmeldenummer: 02022157.8
(22) Anmeldetag: 02.10.2002
(51) Int. Cl.: A61L 9/20, A47L 7/04

(54) **Verfahren und Vorrichtung zur Luft- und Raumreinigung**

(30) Priorität: 02.10.2001 DE 10148718
(71) Anmelder: Bernd Döring Verkaufsdirektion GmbH, 63546 Hammersbach (DE)
(72) Erfinder: Döring, Bernd, 63456 Hammersbach (DE)
(74) Vertreter: Stoffregen, Hans-Herbert, Dr. Dipl.-Phys.

(57) **Zusammenfassung**

Die Erfindung bezieht sich auf ein Verfahren sowie eine Vorrichtung zur Luft- und Raumreinigung, wobei verunreinigte Luft und/oder Flüssigkeit durch eine Sangeinheit (12) über eine Einlassöffnung (14) in ein mit Flüssigkeit (16) gefülltes Behältnis (18) eingesaugt wird und wobei ein Luft- und/oder Flüssigkeitsstrom die Flüssigkeit (16) durchsetzt und von Verunreinigungen wie Schmutzteilchen befreit wird und wobei die angesaugte Luft anschließend gegebenenfalls nach Durchströmen eines Zentrifugalabscheiders über eine Auslassöffnung (22) abgeführt wird. Zur Verbesserung der Reinigungseigenschaften ist vorgesehen, dass durch den aus der Flüssigkeit (16) austretenden Luft- und/oder Flüssigkeitsstrom an der Oberfläche (38) der Flüssigkeit (16) ein Finssigkeitsdunst erzeugt wird und dass der Flüssigkeitsdunst zum Abtöten von in dem Luft- und/oder Flüssigkeitsstrom enthaltenen Keimen einer UV-Strahlung ausgesetzt wird.

## Beschreibung

Die Erfindung bezieht sich auf ein Verfahren zur Luft- und Raumreinigung, wobei ein Medium durch eine Saugeinheit über eine Einlassöffnung in ein mit Flüssigkeit gefülltes Behältnis eingesaugt und entlang einer UV-bestrahlten Flüssigkeitsoberfläche geführt und von Verunreinigungen wie Schmutzteilchen und Keimen befreit wird und wobei die angesaugte Luft anschließend gegebenenfalls nach Durchströmen eines Zentrifugalabscheiders über eine Auslassöffnung abgeführt wird sowie auf eine Vorrichtung zur Durchführung des Verfahrens.

In der JP 08108028 A, die als nächstliegender Stand der Technik angegeben wird, ist eine Vorrichtung zur Reinigung von Luft beschrieben. Dabei wird die durch Schmutzpartikel verunreinigte Luft über ein Gebläse durch einen Ansatzstutzen in die Vorrichtung geleitet und durch eine Auslassöffnung wieder an die Umgebung abgegeben. Die dabei zwischen entströmender Luft und dem Wasserbad wirksame Fläche zur Aufnahme der Schmutzpartikel ist auf die Wasserbadoberfläche begrenzt und somit in ihrer Reinigungswirkung eingeschränkt. Die durch eine UV-Quelle bestrahlte Oberfläche ist ebenfalls auf die Fläche des Wasserbades beschränkt. Schließlich ist die bekannte Vorrichtung nur zur Reinigung von angesaugter Flüssigkeit geeignet. Wenn das die verunreinigte Luft aufnehmende Wasser verschmutzt ist, wird über einen Tank frisches Wasser zugeführt. Das die Schmutzpartikel aufnehmende Wasser und das frische Wasser werden zur Abtötung von Keimen mit einer UV-Lampe bestrahlt.

In der DE 30 09 365 A1 wird ein Staubsauger beschrieben, der neben einem Staubbeutel einen Nachfilter zum Abtöten von Keimen aufweist. Der Nachfilter besteht dabei in einem Ausführungsbeispiel aus einer Anzahl von UV-Strahlern, die längs gerichtet um einen Kanal angeordnet sind. Ein Wasserbad zur Aufnahme der Schmutzpartikel ist nicht vorgesehen.

In der DE 43 33 831 C2 wird eine Vorrichtung zur Beseitigung von Allergenen, insbesondere von Hausstaubmilben beschrieben. Die Vorrichtung umfasst einen eine Ansaugöffnung aufweisenden Staubsauger, wobei als keimtötende Vorrichtung eine UV-Lichtquelle dem Staubsauger zugeordnet ist, die in ihrer Betriebsdauer zeitlich variierbar ist. Die UV-Lichtquelle befindet sich in einem frei beweglichen UV-Handstrahler oder in einem UV-Standgerät und ist nicht in dem Staubsauger integriert.

In der JP 2000 070644 A ist ein Luftreiniger für Automobile beschrieben, bei dem die Luft eines Fahrzeuginnenraums über einen Einlass in ein Gehäuse gesaugt und die von Staub oder ähnlichem zu reinigende Luft gemeinsam mit Wasser über Düsen versprüht wird. Dieser Sprühnebel wird zum Abtöten von Keimen mit einer UV-Lampe bestrahlt. Die gereinigte Luft wird anschließend in den Fahrzeuginnenraum abgegeben. Die Versprühung der zu reinigenden Luft zusammen mit Wasser über Düsen ist relativ aufwendig und kostspielig.

In der Produktbeschreibung "Modell ORKY WFF&D", 08/98, wird ein Luft- und Reinigungssystem beschrieben, bei dem verunreinigte Luft über eine Saugeinrichtung angesaugt wird, wobei der Luftstrom ein Wasserbad durchquert, so dass die in der verunreinigten Luft enthaltenen Staubpartikel in der Flüssigkeit wie beispielsweise Wasser aufgenommen werden. Anschließend durchquert die angesaugte Luft einen Zentrifugalabscheider, in dem Flüssigkeit aus dem Luftstrom ausgeschieden wird, so dass die gereinigte Frischluft anschließend über eine Luftaustrittsöffnung an die Umgebung abgegeben wird.

Untersuchungen haben jedoch ergeben, dass das Waschwasser sowie auch die von der Vorrichtung abgegebene Luft durch Keime verunreinigt sein können.

Davon ausgehend liegt der vorliegenden Erfindung das Problem zu Grunde, eine Vorrichtung der zuvor genannten Art dahingehend weiterzubilden, dass die Wirkung der UV-Strahlung zur Reinigung des Flüssigkeitsbades und/oder des verunreinigten angesaugten Mediums verbessert wird.

Das Problem wird verfahrensmäßig u. a. dadurch gelöst, dass das aus verunreinigter Luft und/oder Flüssigkeit gebildete Medium unterhalb einer Flüssigkeitsoberfläche eingesaugt wird und die Flüssigkeit derart durchströmt, dass durch den aus der Flüssigkeit austretenden Luft- und/oder Flüssigkeitsstrom an der Flüssigkeitsoberfläche eine Verwirbelung stattfindet und ein Flüssigkeitsdunst erzeugt wird, und dass der Flüssigkeitsdunst zum Abtöten von in dem Luft- und/oder Flüssigkeitsstrom enthaltenden Keimen der UV-Strahlung ausgesetzt wird.

Dem erfindungsgemäßen Verfahren liegt der Vorteil zu Grunde, dass die an der Flüssigkeitsoberfläche entstehende Verwirbelung und der daraus entstehende Flüssigkeitsdunst ausgenutzt werden, um eine effektive UV-Bestrahlung durchzuführen, da durch den Flüssigkeitsdunst die wirksame Oberfläche insgesamt vergrößert wird.

Gemäß einer erfindungsgemäßen Vorrichtung ist vorgesehen, dass die Einlassöffnung unterhalb der Flüssigkeitsoberfläche angeordnet ist, so dass das aus verunreinigter Luft und/oder Flüssigkeit gebildete Medium die Flüssigkeit durchsetzt und beim Austreten des Luft- und/oder Flüssigkeitsstroms an der Flüssigkeitsoberfläche eine Verwirbelung erzeugbar ist, durch die ein Flüssigkeitsdunst erzeugt wird, dass die UV-Quelle zum Abtöten von in dem Luft- und/oder Flüssigkeitsstrom enthaltenen Keimen derart angeordnet ist, dass der beim Austreten des Luft- und/oder Flüssigkeitsstromes an der Oberfläche der Flüssigkeit entstehende Flüssigkeitsdunst der UV-Strahlung ausgesetzt ist.

Die erfindungsgemäße Vorrichtung zeigt gegenüber dem Stand der Technik den Vorteil, dass durch die Beaufschlagung der Flüssigkeitsoberfläche mit UV-Licht einerseits die in der Flüssigkeit und andererseits in der von der Vorrichtung angesaugten Luft enthaltenen Keime abgetötet werden. Durch die Luftströmung der eingesaugten Luft durch die Flüssigkeit entsteht an der Flüssigkeitsoberfläche ein Sprudelvorgang und oberhalb der Flüssigkeitsoberfläche ein Flüssigkeitsdunst, der unmittelbar mit UV-Licht beaufschlagt wird, so dass in dem Dunst enthaltene Keime abgetötet werden können.

Eine bevorzugte Ausführungsform zeichnet sich dadurch aus, dass die UV-Quelle an einer der Flüssigkeitsoberfläche zugewandten Unterseite eines das Behältnis verschließenden Deckels angeordnet ist. Hierdurch ergibt sich der Vorteil, dass bereits vorhandene Einrichtungen des Systems zum Einbau der UV-Quellen verwendet werden. Durch die Anordnung der UV-Quelle oberhalb der Flüssigkeitsoberfläche wird aufgrund der aufsprudelnden Flüssigkeit ein intensiver Kontakt der Flüssigkeit bzw. des Flüssigkeitsdampfes mit den UV-Strahlen erreicht.

Eine weitere vorteilhafte Ausführungsform zeichnet sich dadurch aus, dass die UV-Quelle im Bereich einer unterhalb der Flüssigkeitsoberfläche angeordneten Luftaustrittsöffnung angeordnet ist, da in diesem Bereich die maximale Verwirbelung der Flüssigkeit durch aufsteigende Luftblasen erfolgt.

Die UV-Quelle kann als UV-Leuchtstofflampe ausgebildet sein, die an einer Unterseite des Deckels angeordnet ist. Dabei können auch mehrere UV-Quellen entlang des die Unterseite des Deckels begrenzenden Umfangs angeordnet sein, um die Wirkung der UV-Strahlung zu verbessern. Selbstverständlich besteht auch die Möglichkeit, dass die UV-Quelle als ringförmige UV-Leuchtstofflampe ausgebildet ist.

Vorzugsweise ist an der Unterseite des Deckels eine Aufnahme integriert, in der die UV-Quelle aufgenommen ist. Dabei steht die UV-Quelle gegenüber einer von der Unterseite aufgespannten Ebene hervor, um die wirksame Oberfläche zu vergrößern, d. h. eine effektive Strahlungsleistung abgeben zu können.

Eine weitere vorteilhafte Ausführungsform zeichnet sich dadurch aus, dass die UV-Quelle einen Ansaugstutzen des Zentrifugalabscheiders umgibt, so dass der mit Wasserpartikeln angereicherte Luftstrom effektiv mit UV-Strahlen beaufschlagt werden kann.

Weitere Einzelheiten, Vorteile und Merkmale der Erfindung ergeben sich nicht nur aus den Ansprüchen, den diesen zu entnehmenden Merkmalen - für sich und/oder in Kombination, sondern auch aus der nachfolgenden Beschreibung eines den Zeichnungen zu entnehmenden bevorzugten Ausführungsbeispiels.

Es zeigen:
- Fig. 1: eine Vorrichtung zur Reinigung von verunreinigter Luft, umfassend eine als Deckel ausgebildete Saugeinheit zum Verschließen eines Flüssigkeitsbehältnisses.
- Fig. 2: eine Unterseite des Deckels mit integrierten UV-Quellen,
- Fig. 3: eine UV-Quelle als UV-Leuchtstoffröhre und
- Fig. 4: einen elektrischen Schaltkreis zur Ansteuerung der UV-Quellen.

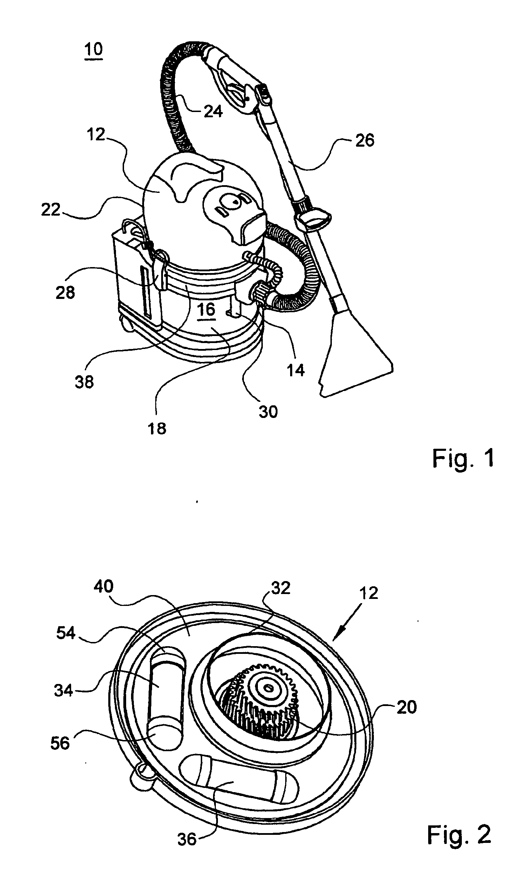

Fig. 1 zeigt eine Explosionsdarstellung einer Vorrichtung 10 zur Luft- und Raumreinigung. Die Vorrichtung 10 umfasst eine Saugeinheit 12, mit der verunreinigte Luft im Staubsaugerbetrieb oder ein Luft-/Flüssigkeitsgemisch im Nasssaugerbetrieb über eine Einlassöffnung 14 in ein mit Flüssigkeit 16 gefülltes Behältnis 18 eingesaugt wird. Die Saugeinheit 12 ist im dargestellten Ausführungsbeispiel mit einem Zentrifugalabscheider 20 ausgestattet, der die an die Umgehung abzugebende Luft von Wasser trennt und gereinigte Luft über eine Auslassöffnung 22 an die Umgebung freigibt. Im dargestellten Ausführungsbeispiel ist die Lufteinlassöffnung 14 über einen Schlauch 24 mit einer Saugdüse 26 verbunden. Im Folgenden wird der Staubsaugerbetrieb näher beschrieben.

Die als Deckel ausgebildete Saugeinheit 12 ist über Spannelemente 28 mit dem Behältnis 18 wasser- und luftdicht verbindbar. Sofern durch die Saugeinrichtung 12 im Innern des Behältnisses 18 ein Unterdruck erzeugt wird, wird Außenluft über die Saugdüse 26 in den Verbindungsschlauch 24 durch die Einlassöffnung 14 angesaugt und wird über einen Führungskanal 30 unterhalb der Wasseroberfläche durch eine Austrittsöffnung 31 freigegeben, so dass eine Luftströmung durch die Flüssigkeit 16 wie Wasser erzeugt wird. Durch den Kontakt der verunreinigten Luft mit dem Wasser 16 werden Verunreinigungen wie Schmutz-/Staubpartikel von dem Wasser aufgenommen und in diesem gelöst. Die in dem Wasser 16 aufsteigende Luft gelangt sodann in den Zentrifugalabscheider 20 und wird in diesem von Wasserpartikeln gelöst, die an einer Abscheidewand 32 zurück in des Behältnis 18 tropfen. Die so gereinigte Luft gelangt über die Auslassöffnung 22 an die Umgebung.

Zur weiteren Reinigung der abgegebenen Luft sowie der in dem Behältnis 18 aufgenommenen Flüssigkeit 16 ist vorgesehen, dass die Vorrichtung 10 zumindest eine UV-Quelle 34, 36 anfweist, über die eine Oberfläche 38 der Flüssigkeit 16 mit UV-Strahlen beaufschlagt werden kann. Gemäß einer bevorzugten Ausführungsform, die in Fig. 2 dargestellt ist, sind die UV-Quellen 34, 36 an einer Unterseite 40 der Saugeinheit 12 angeordnet. Dabei sind die UV-Quellen 34, 36 im Wesentlichen im Bereich der Einlassöffnung 14 angeordnet, so dass eine besonders effektive Bestrahlung der im Bereich der Einlassöffnung 14 aufgewirbelten Flüssigkeit und austretenden Luft erfolgen kann.

Selbstverständlich kann die UV-Quelle 34, 36 auch als umlaufende ringförmige UV-Leuchtstoffröhre ausgebildet sein, so dass ein Ringstrahler entsteht, der die gesamte Oberfläche 38 der Flüssigkeit bestrahlt.

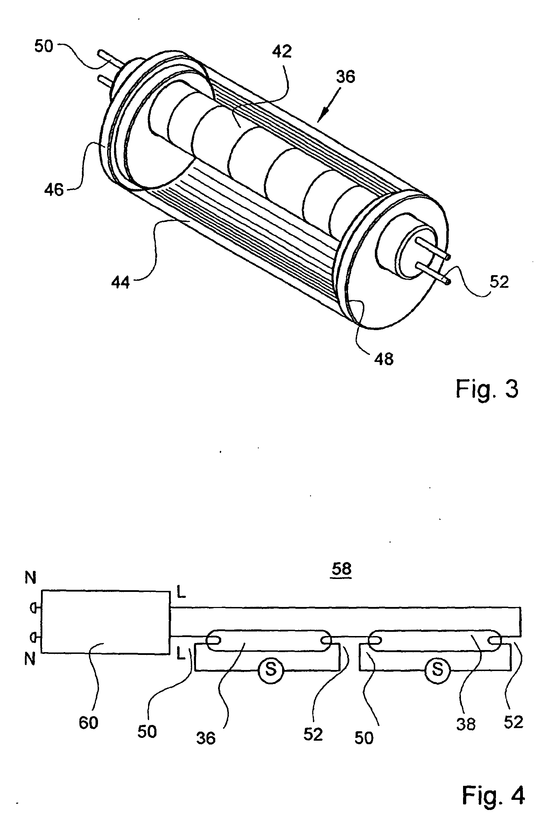

Die UV-Quellen 34, 36 umfassen gemäß Fig. 3 eine Leuchtstoffröhre 42, die von einem Quarzglaszylinder 44 ummantelt ist, der stirnseitig mittels Verschlusselementen 46, 48 vor Feuchtigkeit abgedichtet ist. Die Anschlüsse 50, 52 der Leuchtstoffröhre 42 sind im eingebauten Zustand ebenfalls von Abdeckelementen 54, 56, die integraler Bestandteil der Unterseite 40 des Deckels 12, sind, abgedeckt.

Eine elektrische Schaltung 58 zum Betrieb der UV-Strahler 36, 38 ist beispielhaft in Fig. 4 dargestellt. Die Schaltung 58 ist als Tandem-Schaltung für zwei Leuchtstoffröhren ausgebildet, die eine Leistung im Bereich von 2 bis 50 Watt, vorzugsweise 4 bis 10 Watt aufweisen. Die Leuchtstoffröhren 36, 38 werden von einer Steuerschaltung 60 mit Energie versorgt.

Durch die erfindungsgemäße Ausführungsform und das Verfahren wird erreicht, dass die in der Flüssigkeit 16 und/oder in der verunreinigten Luft und/oder dem verunreinigten Luft-/Flüssigkeitsgemisch enthaltenen Keime abgetötet werden und dass eine im Wesentlichen keimfreie Abluft aus der Vorrichtung 10 über die Auslassöffnung 22 abgegeben wird.

## Patentansprüche

1. Verfahren zur Luft- und Raumreinigung, wobei ein verunreinigtes Medium durch eine Saugeinheit (12) über eine Einlassöffnung (14) in ein mit Flüssigkeit (16) gefülltes Behältnis (18) eingesaugt und entlang einer UV-bestrahlten Flüssigkeitsoberfläche (38) geführt und von Verunreinigungen wie Schmutzteilchen und Keimen befreit wird und wobei das angesaugte Medium anschließend gegebenenfalls nach Durchströmen eines Zentrifugalabscheiders über eine Auslassöffnung (22) abgeführt wird,
**dadurch gekennzeichnet,**
**dass** das aus verunreinigter Luft und/oder Flüssigkeit gebildete Medium unterhalb einer Flüssigkeitsoberfläche (38) eingesaugt wird und die Flüssigkeit (16) derart durchströmt, dass durch den aus der Flüssigkeit (16) austretenden Luft- und/oder Flüssigkeitsstrom an der Flüssigkeitsoberfläche (38) eine Verwirbelung stattfindet und ein Flüssigkeitsdunst erzeugt wird, und dass der Flüssigkeitsdunst zum Abtöten von in dem Luft- und/oder Flüssigkeitsstrom enthaltenden Keimen der UV-Strahlung ausgesetzt wird.

2. Vorrichtung (10) zur Luft und Raumreinigung, umfassend eine Saugeinheit (12), mit der ein verunreinigtes Medium über eine Einlassöffnung (14) in ein mit Flüssigkeit (16) gefülltes Behältnis (18) eingesaugt und entlang einer mit einer UV-Quelle (34) bestrahlten Flüssigkeitsoberfläche (38) geführt und von Verunreinigungen wie Schmutzteilchen und Keimen befreit wird, und wobei die angesaugte Luft anschließend gegebenenfalls nach Durchströmen eines Zentrifugalabscheiders (20) über eine Auslassöffnung (22) abgeführt wird,
**dadurch gekennzeichnet,**
**dass** die Einlassöffnung (14) unterhalb der Flüssigkeitsoberfläche (38) angeordnet ist, so dass das aus verunreinigter Luft und/oder Flüssigkeit gebildete Medium die Flüssigkeit durchsetzt und beim Austreten des Luft- und/oder Flüssigkeitsstroms an der Flüssigkeitsoberfläche (38) eine Verwirbelung erzeugbar ist, durch die ein Flüssigkeitsdunst erzeugt wird, dass die UV-Quelle (34, 36) zum Abtöten von in dem Luft- und/oder Flüssigkeitsstrom enthaltenen Keimen derart angeordnet ist, dass der beim Austreten des Luft- und/oder Flüssigkeitsstromes an der Oberfläche der Flüssigkeit (16) entstehende Flüssigkeitsdunst der UV-Strahlung ausgesetzt ist.

3. Vorrichtung nach Anspruch 2,
**dadurch gekennzeichnet,**
**dass** die vorzugsweise als UV-Leuchtstofflampe ausgebildete UV-Quelle (34, 36) an einer der Flüssigkeitsoberfläche (38) zugewandten Unterseite (40) eines das Behältnis (18) verschließenden Deckels (12) angeordnet, vorzugsweise in die Unterseite (40) des Deckels (12) integriert ist.

4. Vorrichtung nach Anspruch oder 3,
**dadurch gekennzeichnet,**
**dass** die UV-Quelle (34, 36) im Bereich einer unterhalb der Flüssigkeitsoberfläche (38) angeordneten Luftaustrittsöffnung (30) angeordnet ist.

5. Vorrichtung nach zumindest einem der vorhergehenden Ansprüche,
**dadurch gekennzeichnet,**
**dass** die UV-Quelle (34, 36) entlang des die Unterseite (40) des Deckels (12) begrenzenden Umfangs angeordnet ist.

6. Vorrichtung nach zumindest einem der vorhergehenden Ansprüche,
**dadurch gekennzeichnet,**
**dass** die UV-Quelle (34, 36) als ringförmige oder stabförmige UV-Leuchtstofflampe ausgebildet ist.

7. Vorrichtung nach zumindest einem der vorhergehenden Ansprüche,
**dadurch gekennzeichnet,**
**dass** die UV-Quelle (34, 36) gegenüber einer von der Unterseite (40) aufgespannten Ebene hervorsteht.

8. Vorrichtung nach zumindest einem der vorhergehenden Ansprüche,
**dadurch gekennzeichnet,**
**dass** die UV-Quelle einen Ansaugstutzen des Zentrifugalabscheiders (20) umgibt.
